# EUROPEAN PATENT APPLICATION

(11) **EP 0 680 971 A1**
(43) Date of publication of application: **08.11.1995**
(21) Application number: 95109112.3
(22) Date of filing: 29.09.1989
(51) Int. Cl.: C07K 5/06

(54) **Dry crystals of alpha-L-aspartyl-L-phenylalanine methyl ester having improved solubility**

(30) Priority: 03.10.1988 JP 249682/88
(62) Divisional of application: 89118040.8
(71) Applicant: Ajinomoto Co., Inc., Tokyo 104 (JP)
(72) Inventor: Ohura, Harutoshi, c/o Tokai Plant Ajinomoto, Yokkaichi-shi, Mie-ken (JP); Yasaki, Akihiko, c/o Central Research Lab., Kawasaki-ku, Kawasaki-shi, Kanagawa-ken (JP); Kishimoto, Shinichi, c/o Central Research Lab., Kawasaki-ku, Kawasaki-shi, Kanagawa-ken (JP); Naruse, Masayoshi, c/o Central Research Lab., Kawasaki-ku, Kawasaki-shi, Kanagawa-ken (JP)
(74) Representative: Strehl Schübel-Hopf Groening & Partner

(57) **Abstract**

Dry I_{B} type crystals of α-L-aspartyl-L-phenylalanine methyl ester having improved solubility may be obtained by a process, wherein
(a) the crystallization of α-L-aspartyl-L-phenylalanine methyl ester is carried out without causing a forced liquid flow in the crystallization medium,
(b) the formed crystals are separated from the crystallization medium and subjected to a dewatering treatment to obtain wet crystals having a water content of not more than 50 wt.%, based on the overall weight of the wet crystals of α-L-aspartyl-L-phenylalanine methyl ester, and
(c) the obtained wet crystals having a water content of not more than 50 wt.% are continuously air dried by a pneumatic conveyor dryer with hot air.

## Description

The present invention relates to dry crystals of α-L-aspartyl-L-phenylalanine methyl ester (hereafter simply referred to as aspartame) having improved solubility.

Aspartame is a refreshing sweetener which has a minimized bitter taste or aftertaste generally involved in a highly sweet sweetener. It has thus found widely spread use as a low calorie sweetener. In terms of physical properties, however, it is disadvantageous that aspartame has a poor dispersibility and solubility in water.

In order to improve the dispersibility and solubility of aspartame several approaches have been made to modify aspartame preparations, for example by forming granules, preparing effervesent powders or tablets and the like by adding excipients and disintegrators to the preparations. The copresence of additional substances, such as excipients, often causes trouble depending on the final use of the aspartame. It is therefore a problem to be solved to provide aspartame having high purity, but additionally good solubility and dispersibility in water and other liquids

In an attempt to improve the solubility of aspartame while maintaining the purity, there has been developed a method which comprises spray-drying aspartame in its slurry state (Japanese Patent Publication No. 58-20588), a method which comprises adding water to aspartame to a specific water content and granulating the aspartame (Japanese Patent Application Laid-Open No. 59.95862). With respect to the solubility of aspartame crystals per se, I_{B} type crystals disclosed in Japanese Patent Application Laid-Open No. 59-17244 have good solubility as dry crystals.

Although, it is desirable to prepare pure I_{B} type crystals in an industrial scale, there has not yet been found any satisfactory process for obtaining I_{B} type crystals having high purity.

It has been found that in order to prepare I_{B} type crystals of aspartame having high purity in which excipients, foaming agents, etc. are absent, wet crystals of aspartame may be dried at a relatively low temperature below 80° C.

However, if it is attempted to dry wet crystals of aspartame at a low temperature below 80° C, a long period of time is required. Such a procedure is not an efficient drying method from the industrial viewpoint. Further in order to obtain I_{B} type crystals in high purity, complicated operation control is also required. From technical and economical viewpoints, it is thus an important problem to develop a process for continuously preparing I_{B} type crystals having excellent solubility in a short period of time.

As a result of extensive investigations in order to solve the foregoing problem, the inventors have found that dry crystalline α-L-aspartyl-L-phenylalanine methyl ester having improved solubility is obtainable by a process wherein
(a) the crystallization of α-L-aspartyl-L-phenylalanine methyl ester is carried out without causing a forced liquid flow in the crystallization medium,
(b) the formed crystals are separated from the crystallization medium, and
(c) wet crystals having a water content of not more than 50 wt.%, based on the overall weight of the wet crystals of α-L-aspartyl-L-phenylalanine methyl ester, are continuously air dried by a pneumatic conveyor dryer with hot air at 80 to 200° C until the water content has reached 2 to 6 wt.%.

According to the present invention, it is possible to control the water content of the formed aspartame crystals to a low water content by applying non-stirring crystallization conditions. By drying the obtained wet crystals with hot air at 80 to 200° C while supplying and preferably recycling the hot air, aspartame in the form of I_{B} type crystals having good solubility can be readily prepared on an industrial scale.

In order to obtain the aspartame wet crystals by crystallization without stirring according to the present invention, one may use the method for crystallization disclosed in Japanese Patent Application JP-A-58-177952. More specifically, the method comprises a step of cooling by conductive heat transfer without applying forced flow, such as mechanical stirring etc., in the crystallization of aspartame from an aqueous solution of aspartame in such a way that the amount of the crystallized solid phase is approximately 10 g or more per liter of solvent after the cooling. By this method a pseudo solid phase is formed. After the pseudo solid phase has been formed, the system is subjected to solid-liquid separation as it is; or if necessary, after further cooling, the solid-liquid separation is performed. Thus, the aspartame wet crystals can be obtained through crystallization without stirring.

The aspartame crystals obtained by the non-stirring crystallization are composed of bundles of fine needles, that is bundle-like crystals apparently forming one crystal mass and which show very good dehydration property in the solid-liquid separation.

If, on the other hand, the crystallization of aspartame from an aqueous solution is carried out by cooling with stirring, that is in a process which comprises a step of performing forced flow such as by mechanical stirring, which has been the usual industrial method, it has been found that a slurry-like mixture of mother liquor and crystals is formed, in which the obtained aspartame crystals are needles which after filtration still have a high water content of approximately 75 %. Even after centrifugation the water content is slightly over 50 %. Therefore, when the wet aspartame crystals obtained by crystallization under stirring are fed to a dryer, such as a jet air dryer, it is unavoidable that the wet crystals will adhere to the inner wall of the dryer resulting in overdrying and scorching of the adhering crystals. This process therefore results not only in procedural difficulties, such as breakdown by clogging of the apparatus, but also to an inferior quality of the obtained product. Since furthermore the dispersibility of the obtained crystals in the air flow is poor in the drying process, the product shows the tendency of forming large agglomerated masses. Furthermore, in the step of feeding the wet crystals to the dryer, the crystals tend to adhere to the feeding screw, already leading to difficulties during the feeding step.

To the contrary, the aspartame wet crystals provided by crystallization without stirring can be fed to an air flow drier after the centrifugation as they are, since the water content is not more than 50 wt.% after centrifugation, generally 20 to 50 % and in the case of a further centrifugation, approximately 10 to 20 %. Even in this case, the foregoing problems are not caused but the drying can be performed smoothly and efficiently to prepare the I_{B} type crystals of aspartame in high purity. The aspartame wet crystals also include granules in a wet crystalline state, which are obtained by extruding the wet crystals through a screen.

Suitable devices for continuously drying while flowing air in the process of the present invention are conventional dryers used for continuous air drying, known as pneumatic conveyor dryers, which are described e.g. in Perry's Chemical Engineer's Handbook, 6th Edition, 1984, McGraw-Hill, pages 20-51 to 20-54.

When aspartame is dried at a high speed, a part of aspartame tends to be converted into diketopiperazine derivatives which are not sweet at all, resulting in a loss of the overall sweetness. According to the present invention, the drying is continuously carried out by means of a pneumatic conveyor dryer while flowing hot air and completed in a period as short as within about one minute. Even when using hot air at high temperatures of 80 to 200 °C, desirably 130 to 160 °C, the short drying period suppresses the conversion to diketopiperazine derivatives, and the formation of type II crystals described in Japanese Patent Application Laid-Open no. 59-172444 is minimized. The drying in the pneumatic conveyor dryer is carried out at an inlet temperature of 80 to 200 °C, preferably 130 to 160 °C, and an outlet temperature of 50 to 180 °C, preferably 70 to 130 °C. Accordingly, I_{B} type crystals of aspartame can be prepared stably with extremely good efficiency. In case that the temperature of hot air is below 80°C, a long period of time is required for the drying. Conversely, when the temperature is higher than 200°C, the rate of causing rearrangement to type II crystals increases. Therefore, temperatures outside the above range are not preferred for industrially preparing I_{B} type crystals stably.

Dry crystals (I_{B} type crystals) of aspartame having a water content of approximately 2 to 6% can be obtained by the continuous drying while supplying hot air. The obtained I_{B} type crystals of aspartame have good solubility and show excellent powder properties, such as minimized scattering properties in handling.

The present invention is described in more detail by referring to the examples below.

### Example 1

A raw aqueous solution, 380 liters, in which 17.7 kg of aspartame (55° C, initial aspartame concentration of 4.4 wt%) had been dissolved was charged to a crystallization device having a diameter of 400 mm equipped with a jacket and having a cooling plate in the inside. A coolant having a temperature of 0° C was cycled through the jacket and the cooling plate to cool the solution for over 3 hours. About an hour after, the solution as a whole became a pseudo solid phase. The aspartame crystals in the pseudo solid phase were brought into a receiving tank equipped with a stirrer and homogenized to form a slurry. The slurry was further cooled (from 16°C to 7°C) in the receiving tank. The thus obtained slurry was filtered and dehydrated with a centrifuging device having a diameter of 91.5 cm (36 inches) to give aspartame wet crystals having a water content of 30 %. The wet crystals obtained by such non-stirring crystallization were continuously fed to a Micron drier (manufactured by Hosokawa Micron Co., Ltd.) using a screw feeder. Drying conditions:

| | |
|---|---|
| Temperature of hot air at drier inlet | 135°C |
| Temperature of waste air at drier outlet | 104°C |

The amount of the I_{B} type crystals after drying was determined by the ratio of characteristic peaks of the I_{B} type crystals to II type crystals in powdery X ray diffraction patterns (the same also applies to the following examples). Results:

| | |
|---|---|
| Water content | 2.6% |
| Amount of I_{B} type crystals | 95% |
| Diketopiperazine derivatives | less than 0.05 % |

### Example 2

Aspartame wet crystals (water content of 30% based on the weight of aspartame wet crystals) were obtained by non-stirring crystallization in a manner similar to Example 1. The wet crystals were fed to the same Micron drier as in Example 1 and air dried under drying conditions described below. Drying conditions:

| | |
|---|---|
| Temperature of hot air at drier inlet | 160°C |
| Temperature of waste air at drier outlet | 109°C |

### Results:

| | |
|---|---|
| Water content | 2.4% |
| Amount of I_{B} type crystals | 87% |
| Diketopiperazine derivatives | less than 0.05 |

## Claims

1. Dry I_{B} type crystals of α-L-aspartyl-L-phenylalanine methyl ester having improved solubility obtainable by a process wherein
(a) the crystallization of α-L-aspartyl-L-phenylalanine methyl ester is carried out without causing a forced liquid flow in the crystallization medium,
(b) the formed crystals are separated from the crystallization medium and subjected to a dewatering treatment to obtain wet crystals having a water content of not more than 50 wt.%, based on the overall weight of the wet crystals of α-L-aspartyl-L-phenylalanine methyl ester, and
(c) the obtained wet crystals having a water content of not more than 50 wt.% are continuously air dried by a pneumatic conveyor dryer with hot air at an outlet temperature or 50 to 180 °C until the water content has reached 2 to 6 wt.%.

2. Dry I_{B} type crystals according to Claim 1, wherein in the process the drying in the pneumatic conveyor dryer is carried out at an inlet temperature of 80 to 200 °C, preferably 130 to 160 °C, and an outlet temperature of 50 to 180 °C, preferably 70 to 130 °C.
